# EUROPEAN PATENT APPLICATION

(11) **EP 4 538 964 A1**
(43) Date of publication of application: **16.04.2025**
(21) Application number: 23203228.4
(22) Date of filing: 12.10.2023
(51) Int. Cl.: G06T 7/00, A61B 6/03, A61B 6/50, A61B 6/00

(54) **DETECTION OF VESSEL ABNORMALITY BASED ON MACHINE LEARNING**

(71) Applicant: Siemens Healthineers AG, 91301 Forchheim (DE)
(72) Inventor: Ayman, Suha, 560072 Bengaluru (IN); Jangra, Ashish, 560100 Bangalore (IN); Khan, Rumman, 560100 Bangalore (IN); Nimmagadda, Raveendra Babu, 522019 Gottipadu, Prathipadu, Guntur (IN); Kaergel, Rainer, 96135 Stegaurach (DE)
(74) Representative: Siemens Healthineers Patent Attorneys

(57) **Abstract**

Techniques for processing one or more computed tomography angiographic images are disclosed. The processing may take place either during or after a computed tomography angiography exam of an anatomical region of interest. The one or more computed tomography angiographic images are processed using at least one machine learning algorithm, and a presence of at least one abnormality associated with one or more vessel segments comprised in the anatomical region of interest is determined, detected, or diagnosed.

## Description

Various examples of the disclosure generally relate to computed tomography angiography (CTA). Various examples specifically relate to processing one or more computed tomography (CT) angiographic images associated with an anatomical region of interest which are acquired during a CTA exam to determine a presence of at least one abnormality associated with one or more vessel segments within the anatomical region of interest.

An aneurysm is an outward bulging of a blood vessel wall due to a localized, abnormal, weak spot in the blood vessel wall. Aneurysms may be a result of a hereditary condition or an acquired disease. Aneurysms are usually detected by medical imaging of blood vessels, using different imaging methods and modalities such as angiography, CT imaging, MR imaging or ultrasound imaging. Aneurysms can be due to a hereditary condition or an acquired disease.

As an example, Kawasaki disease (KD) is seen in children of age 1 to 5 years, and it primarily affects the coronary arteries. The disease causes inflammation in the cardiac arteries. Increased fever and patches on the skin are some of the symptoms of the disease. The disease indicates some potentially risky diseases that can develop in children if the condition is not diagnosed and cured in time.

If KD is left untreated, it can lead to serious complications. Coronary Artery Abnormalities (CAAs) and myocarditis are the major cardiovascular complications of KD. CAAs develop in 15-25% of untreated KD cases. This can be particularly dangerous because it can affect the coronary arteries, causing Coronary Artery Aneurysms to develop.

Coronary Artery Aneurysms require long-term follow-up. The aim is to determine the progression of the Coronary Artery Aneurysm and monitor development of thrombotic and steno-occlusive complications. The American Heart Association (AHA) proposes the application of interval echocardiography, stress echocardiography, and invasive or non-invasive coronary angiography in follow-up of patients with KD.

Echocardiography such as two-dimensional transthoracic echocardiography has been used as the standard diagnostic modality for diagnosing KD in children, and other CAAs during the acute stage and on follow-up. Echocardiography, however, has its own inherent limitations. It is highly operator dependent, requires experience and may not be able to delineate distal segments of coronary arteries and left circumflex artery. Besides, it has poor sensitivity in demonstrating intra-luminal thrombus or arterial stenosis. Furthermore, as the child grows, assessment of coronary arteries becomes more difficult because of a limited acoustic window.

Other imaging techniques, e.g., catheter angiography and Magnetic Resonance Angiography (MRA), can supplement echocardiography. However, there are no clear-cut recommendations or guidelines on their usage particularly in children with KD.

Catheter angiography is an option for evaluation of CAAs, but it is invasive, cannot be repeated at frequent intervals, requires sedation, and is associated with high radiation exposure. It may also fail to detect intramural changes in coronary arteries.

MRA is another possible imaging modality as it has the implicit advantage of zero radiation exposure. However, temporal and spatial resolution of MRA is limited. Further, MRA is technically difficult and time-consuming, and few medical centers have the requisite expertise in performing the procedure, particularly for young children. Higher acquisition time associated with MRA adds to artifacts, especially in younger patients.

Accordingly, there is a need for advanced techniques which mitigate or overcome the above-identified drawbacks or restrictions. There is a need exists for advanced techniques for diagnosing abnormalities associated with one or more vessel segments within an anatomical region of interest, e.g., during the acute stage and/or on follow-up.

This need is met by the features of the independent claims. The features of the dependent claims define embodiments.

A computer-implemented method for processing one or more computed tomography angiographic images associated with an anatomical region of interest is provided. The anatomical region of interest comprises one or more vessel segments. The method comprises obtaining the one or more computed tomography angiographic images. The method further comprises determining, using at least one machine learning algorithm, a presence of at least one abnormality associated with the one or more vessel segments based on the one or more computed tomography angiographic images.

A further computer-implemented method is provided. The method comprises obtaining a first set of computed tomography angiographic images acquired during an exam of an anatomical region of interest comprising one or more vessel segments. The method further comprises obtaining a second set of computed tomography angiographic images acquired during a follow-up exam of the anatomical region of interest. The method also comprises processing the first set and the second set according to the method described above. The method still further comprises establishing a diagnosis associated with the one or more vessel segments based on said processing.

A computing device comprising a processor and a memory is provided. Upon loading and executing program code from the memory, the processor is configured to perform a method for processing one or more computed tomography angiographic images associated with an anatomical region of interest. The anatomical region of interest comprises one or more vessel segments. The method comprises obtaining the one or more computed tomography angiographic images. The method further comprises determining, using at least one machine learning algorithm, a presence of at least one abnormality associated with the one or more vessel segments based on the one or more computed tomography angiographic images.

A computed tomography scanner comprising a computing device is provided. The computing device comprises a processor and a memory. Upon loading and executing program code from the memory, the processor is configured to perform a method for processing one or more computed tomography angiographic images associated with an anatomical region of interest. The anatomical region of interest comprises one or more vessel segments. The method comprises obtaining the one or more computed tomography angiographic images. The method further comprises determining, using at least one machine learning algorithm, a presence of at least one abnormality associated with the one or more vessel segments based on the one or more computed tomography angiographic images.

A computer program product or a computer program or a computer-readable storage medium including program code is provided. The program code can be executed by at least one processor. Executing the program code causes the at least one processor to perform a method for processing one or more computed tomography angiographic images associated with an anatomical region of interest. The anatomical region of interest comprises one or more vessel segments. The method comprises obtaining the one or more computed tomography angiographic images. The method further comprises determining, using at least one machine learning algorithm, a presence of at least one abnormality associated with the one or more vessel segments based on the one or more computed tomography angiographic images.

In particular, the features and advantages described in connection with the computer implemented method according to the invention can also be designed as corresponding subunits of the computing device according to the invention or of the computer program product according to the invention. Conversely, the features and advantages described in connection with the device according to the invention or the computer program according to the invention can also be designed as corresponding process steps of the process according to the invention.

It is to be understood that the features mentioned above and those yet to be explained below may be used not only in the respective combinations indicated, but also in other combinations or in isolation without departing from the scope of the invention.

Independent of the grammatical term usage, individuals with male, female or other gender identities are included within the term.
FIG. 1 schematically illustrates an exemplary geometry of a CT scanner.
FIG. 2 schematically illustrates an exemplary 3D frontal view of a heart and of coronary arteries.
FIG. 3 is a flowchart of a method according to various examples.
FIG. 4 schematically illustrates exemplary slices of CT images.
FIG. 5 schematically illustrates exemplary CTCA images.
FIG. 6 schematically illustrates further exemplary CTCA images.
FIG. 7 schematically illustrates an exemplary CTCA image depicting abnormalities with respective Z-scores and severity.
FIG. 8 is a flowchart of a further method according to various examples.
FIG. 9 schematically illustrates an exemplary workflow according to various examples.
FIG. 10 is a block diagram of a computing device according to various examples.

Some examples of the present disclosure generally provide for a plurality of circuits or other electrical devices. All references to the circuits and other electrical devices and the functionality provided by each are not intended to be limited to encompassing only what is illustrated and described herein. While particular labels may be assigned to the various circuits or other electrical devices disclosed, such labels are not intended to limit the scope of operation for the circuits and the other electrical devices. Such circuits and other electrical devices may be combined with each other and/or separated in any manner based on the particular type of electrical implementation that is desired. It is recognized that any circuit or other electrical device disclosed herein may include any number of microcontrollers, a graphics processor unit (GPU), integrated circuits, memory devices (e.g., FLASH, random access memory (RAM), read only memory (ROM), electrically programmable read only memory (EPROM), electrically erasable programmable read only memory (EEPROM), or other suitable variants thereof), and software which co-act with one another to perform operation(s) disclosed herein. In addition, any one or more of the electrical devices may be configured to execute a program code that is embodied in a non-transitory computer readable medium programmed to perform any number of the functions as disclosed.

In the following, embodiments of the invention will be described in detail with reference to the accompanying drawings. It is to be understood that the following description of embodiments is not to be taken in a limiting sense. The scope of the invention is not intended to be limited by the embodiments described hereinafter or by the drawings, which are taken to be illustrative only.

The drawings are to be regarded as being schematic representations and elements illustrated in the drawings are not necessarily shown to scale. Rather, the various elements are represented such that their function and general purpose become apparent to a person skilled in the art. Any connection or coupling between functional blocks, devices, components, or other physical or functional units shown in the drawings or described herein may also be implemented by an indirect connection or coupling. A coupling between components may also be established over a wireless connection. Functional blocks may be implemented in hardware, firmware, software, or a combination thereof.

Hereinafter, techniques of CTA are described. CTA may be employed to obtain one or more CT angiographic images associated with an anatomical region of interest, e.g., a region of the heart, the brain, the kidney, or the lung.

Significant advancements in CT technology including the availability of increased detector rows and advanced software can facilitate a comprehensive non-invasive evaluation of blood vessels within an anatomical region of interest.

For example, computed tomography coronary angiography (CTCA) can allow a comprehensive non-invasive evaluation of arteries including luminal changes, intramural changes, and/or plaque morphology objectively along the entire course of coronary arteries at a reasonably low radiation dose so that it is possible to delineate the coronary artery anatomy with higher temporal resolution and motion-free images at all heart rates with acceptable radiation exposure.

Accordingly, techniques of CTCA can be used to diagnose abnormalities associated with one or more segments of arteries of the heart. It is possible to overcome the dependency on experienced human operators and allow visualization of deeper cardiac arteries in the distal segments and left circumflex artery by using CTCA.

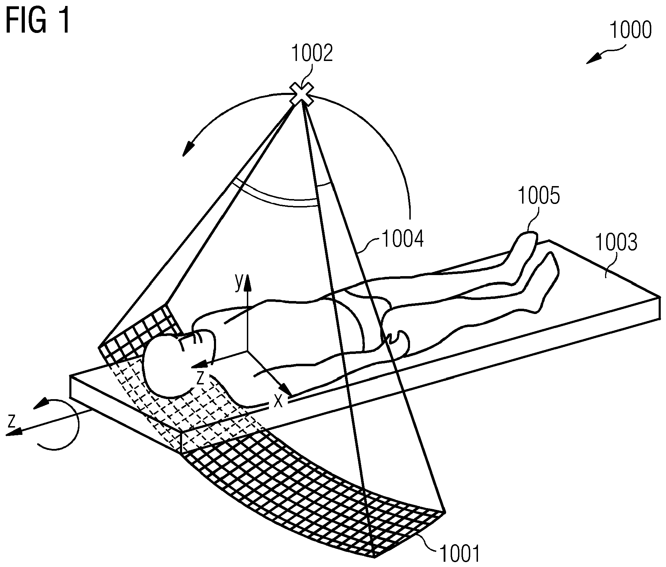

FIG. 1 schematically illustrates an exemplary geometry of a CT scanner 1000. The CT scanner 1000 comprises an x-ray tube 1002, a detector array 1001, a patient table 1003. The x-ray tube 1002 may be a cone-beam x-ray tube emitting an x-ray beam 1004 divergent in and covering an appreciable extent in the longitudinal (z) direction. The detector array 1001 may be a curved detector array having multiple rows of detectors. Both the x-ray tube 1002 and the detector array 1001 may be mounted on a C-arm, U-arm, or O-arm gantry depending on clinical applications ranging from image-guided interventions to diagnostic specialties. The CT scanner 1000 may operate with the patient 1005 stationary on the patient table 1003, and the x-ray tube 1002 together with the detector array 1001 rotate once to acquire a volumetric image. Alternatively or optionally, the CT scanner 1000 may operate using helical acquisition - with exquisitely engineered patient table 1003 for longitudinal (z-direction) translation of the patient during the scan.

According to various examples, the CT scanner 1000 may be a photon-counting CT scanner and may utilize techniques as disclosed in a non-patent literature - Rotzinger, David C., et al. "Performance of spectral photon-counting coronary CT angiography and comparison with energy-integrating-detector CT: objective assessment with model observer." Diagnostics 11.12 (2021): 2376.

Hereinafter, techniques for processing one or more CT angiographic images associated with an anatomical region of interest are disclosed. The one or more CT angiographic images are processed using at least one machine learning algorithm, and a presence of at least one abnormality associated with one or more vessel segments comprised in the anatomical region of interest is determined, detected, or diagnosed.

The processing of the one or more CT angiographic images may take place either during or after a CTA exam of the anatomical region of interest, i.e., either real-time processing or post-processing is possible.

According to various examples, the one or more CT angiographic images may be obtained directly from a CT scanner such as the CT scanner 1000 shown in FIG. 1 or from a database for storing the one or more CT angiographic images acquired by the CT scanner, e.g., a picture archiving and communication system (PACS).

According to various examples, the anatomical region of interest may comprise a region of the heart, the brain, the kidney, or the lung. Hereinafter, the techniques of the disclosure will be described in connection with an anatomical region of the heart.

For example, the anatomical region of interest may comprise a region of the heart of a child and the at least one abnormality comprises one or more aneurysms.

According to various examples, a name (e.g., any one in the column "Abbreviation" in Table 1) of a specific segment of the one or more vessel segments can be determined based on the anatomical region of interest. For example, such an anatomical region of interest may comprise the left or right branch of the coronary vessel tree or a specific vessel section. The anatomical region of interest may be determined based on user input, or on a patient's electronic medical record included in a health information system.

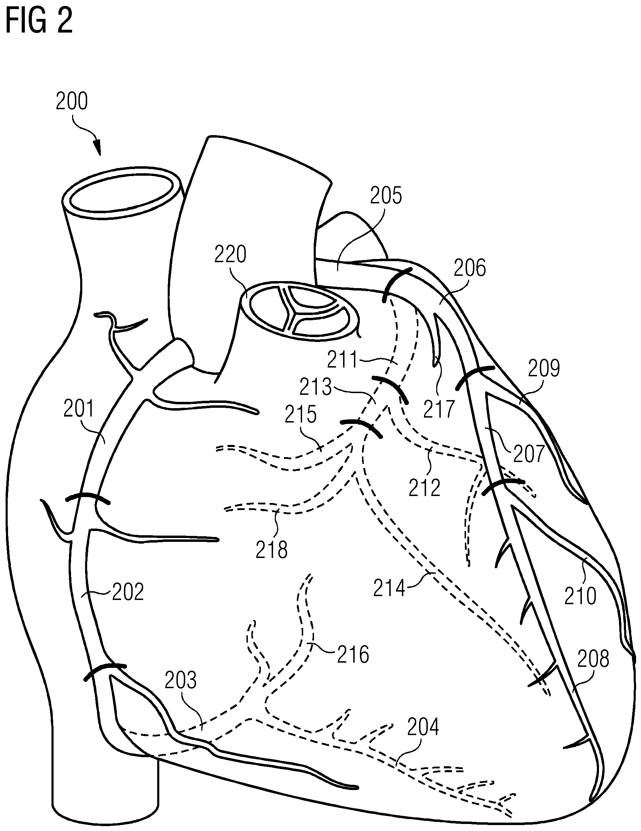

For example, the one or more vessel segments within the anatomical region of interest may be one or more segments of the coronary arteries of the heart. FIG. 2 schematically illustrates an exemplary 3D frontal view 200 of a heart and of coronary arteries. The segments of the coronary arteries in FIG. 2 are based on and numbered according to the vessel segmentation as proposed by the American Heart Association (AHA) and as amended by the Society of Cardiovascular Computed Tomography (SCCT). Table 1 provides a short description of the respective vessel segments as well as an abbreviation for each vessel segment.

**Table 1: Segments of the Coronary Arteries in FIG. 2.**

| **Ref. Sign** | **Vessel Segment** | **Abbreviation** | **Description** |
|---|---|---|---|
| 201 | proximal right coronary artery | pRCA | Ostium of the RCA to one-half the distance to the acute margin of heart |
| 202 | Mid RCA | mRCA | End of pRCA to the acute margin of heart |
| 203 | Distal RCA | dRCA | End of mRCA to origin of the PDA (posterior descending artery) |
| 204 | PDA-R | R-PDA | PDA from RCA |
| 205 | Left main | LM | Ostium of LM to bifurcation of LAD (left anterior descending artery) and LCx (left circumflex artery) |
| 206 | Proximal LAD | pLAD | End of LM to the first large septal or D1 (first diagonal), whichever is most proximal |
| 207 | Mid LAD | mLAD | End of proximal LAD to one-half the distance to the apex |
| 208 | Distal LAD | dLAD | End of mid LAD to end of LAD |
| 209 | D1 | D1 | First diagonal branch D1 |
| 210 | D2 | D2 | Second diagonal branch D2 |
| 211 | Proximal LCx | pCx | End of LM to the origin of the OM1 (first obtuse marginal) |
| 212 | OM1 | OM1 | First OM1 traversing the lateral wall of the left ventricle |
| 213 | Mid and distal LCx | LCx | Traveling in the atrioventricular groove, distal to the OM1 branch to the end of the vessel or origin of the L-PDA |
| 214 | OM2 | OM2 | Second marginal OM2 |
| 215 | PDA-L | L-PDA | PDA from LCx |
| 216 | PLB-R | R-PLB | PLB from RCA |
| 217 | Ramus intermedius | RI | Vessel originating from the left main between the LAD and LCx in case of a trifurcation |
| 218 | PLB-L | L-PLB | PLB from LCx |

In addition to the vessel segments listed in Table 1, FIG. 2 also indicates the aortic valve 220.

According to this disclosure, the at least one machine learning algorithm may comprise one or more of an ensemble learning algorithm, a convolutional neural network, and a deep belief network.

For example, the convolutional neural network may comprise a 3-dimentional U-Net, e.g., that presented in a non-patent literature - Çiçek, Özgün, et al. "3D U-Net: learning dense volumetric segmentation from sparse annotation." Medical Image Computing and Computer-Assisted Intervention-MICCAI 2016: 19th International Conference, Athens, Greece, October 17-21, 2016, Proceedings, Part II 19. Springer International Publishing, 2016.

According to various examples, ensemble learning may be used to process the one or more CT angiographic images and thereby the presence of the at least one abnormality can be determined automatically, precisely, and robustly.

The ensemble learning algorithm for processing the one or more CT angiographic images may be any available algorithm prior to the filing date of this application. For example, the ensemble learning algorithm may be any one described in a non-patent literature - Mienye, Ibomoiye Domor, and Yanxia Sun. "A survey of ensemble learning: Concepts, algorithms, applications, and prospects." IEEE Access 10 (2022): 99129-99149.

In general, ensemble learning algorithms may use multiple base machine learning models to obtain better predictive performance than could be obtained from any of the constituent base machine learning models alone.

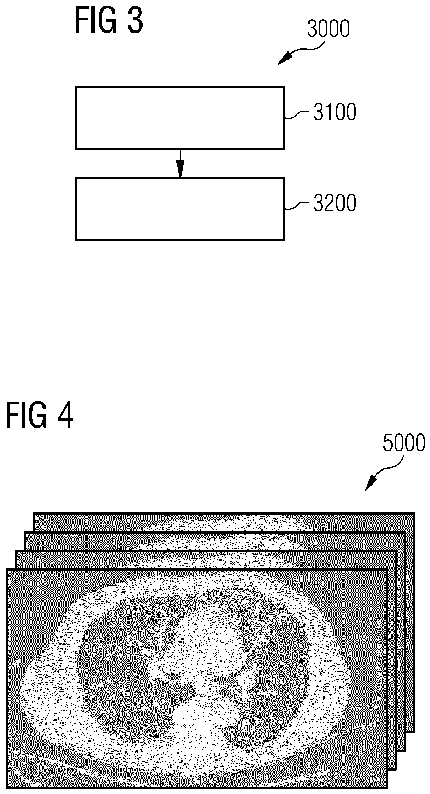

FIG. 3 is a flowchart of a method 3000 according to various examples. The method 3000 pertains to determining, detecting, or diagnosing a presence of at least one abnormality associated with one or more vessel segments comprised in an anatomical region of interest.

The method 3000 may be executed either during or after a CTA exam of the anatomical region of interest, i.e., either real-time processing or post-processing is possible.

The method 3000 according to FIG. 3 may be executed by a computing device comprising at least one processing unit upon loading program code from a memory. Such a computing device may be either embedded in a CT scanner such as the CT scanner 1000 shown in FIG. 1 or connectable to the CT scanner or a PACS. Details of the method 3000 are described below.

Block 3100: obtaining one or more computed tomography angiographic images associated with an anatomical region of interest, wherein the anatomical region of interest comprises one or more vessel segments.

For example, the one or more CT angiographic images may be obtained directly from a CT scanner such as the CT scanner 1000 shown in FIG. 1 or from a database for storing the one or more CT angiographic images acquired by the CT scanner, e.g., a PACS.

According to various examples, the anatomical region of interest may comprise a region of the heart, the brain, the kidney, or the lung. Hereinafter, the techniques of the disclosure will be described in connection with an anatomical region of the heart.

For example, the anatomical region of interest may comprise a region of the heart of a child and the at least one abnormality comprises one or more aneurysms.

According to various examples, each of the one or more vessel segments within (or comprised in) the anatomical region of interest may be either an individual segment or a combination of segments of the coronary arteries of the heart, e.g., as shown in FIG. 2.

For example, the one or more vessel segments may comprise one or more of pLAD 206, mLAD 207, and dLAD 208. Alternatively or optionally, the one or more vessel segments may comprise one more of LAD (i.e., a combination of pLAD 206, mLAD 207, and dLAD 208) and RCA (i.e., a combination of pRCA 201, mRCA 202, and dRCA 203).

FIG. 4 schematically illustrates exemplary slices of CT images 5000 associated with the heart. The slices of CT images 5000 may be obtained using the CT scanner 1000 of FIG. 1 in spatial domain, i.e., x-y plane or axial plane, and orthogonal to the z-axis. The slices of CT images 5000 may be reconstructed by the CT scanner 1000 based on signals detected by the detector array 1001.

After obtaining the slices of CT images 5000, various medical image processing algorithms are available for processing the slices of CT images 5000, e.g., segmenting vessel centerlines and/or visualizing the heart.

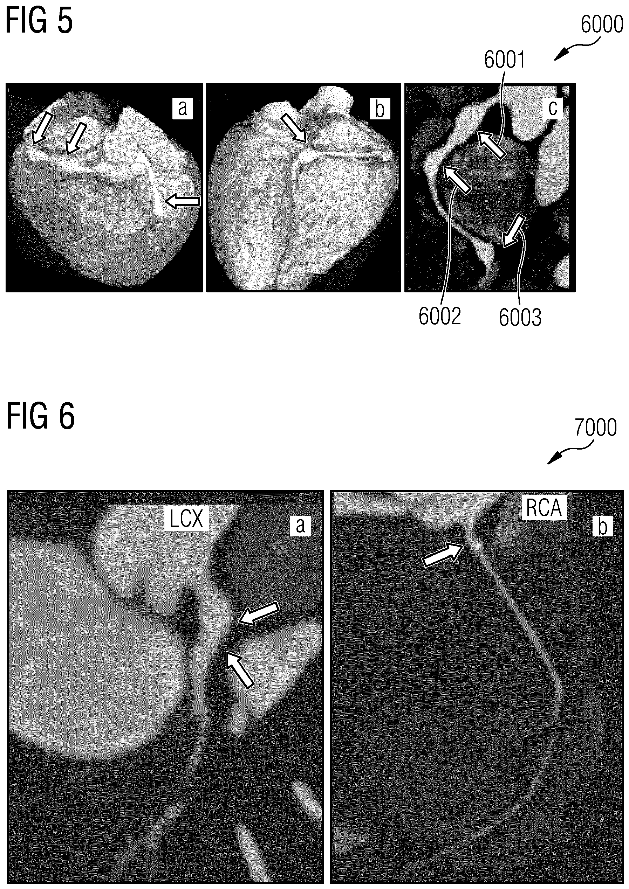

For example, FIG. 5 schematically illustrates exemplary CTCA images 6000. The two images on the left and in the middle, i.e., a and b are respectively exemplary volume-rendered images. The image on the right, i.e., c, is an exemplary curved maximum intensity projection (MIP) image which depicts two fusiform aneurysms 6001 and 6002 in proximal right coronary artery (RCA) and one 6003 in distal RCA at its bifurcation into its posterior descending and postero-lateral branches (thin arrows).

As a further example, FIG. 6 schematically illustrates further exemplary CTCA images 7000. Specifically, FIG. 6 schematically illustrates two exemplary curved maximum intensity projection (MIP) images, i.e., a and b. The left image, i.e., a, depicts fusiform aneurysmal dilation (indicated by arrows) of proximal left circumflex artery (LCX), and the right image, i.e., b, depicts saccular aneurysm (indicated by arrows) of proximal RCA.

The exemplary CTCA images 6000 of FIG. 5 and the further exemplary CTCA images 7000 of FIG. 6 can be obtained by processing the slices of CT images 5000 of FIG. 4, e.g., using various existing medical visualization techniques.

According to various examples, the one or more computed tomography angiographic images may comprise at least one of:
i) one or more slices of CT images reconstructed based on signals detected by the detector array of a CT scanner, e.g., the slices of CT images 5000 of FIG. 4;
*ii)one or more* volume-rendered images, e.g., the two images on the left and in the middle, i.e., a and b, of FIG. 5;
*iii)* one or more curved MIP images, e.g., the image on the right of FIG. 5 and the two images, i.e., a and b, of FIG. 6.

Block 3200: determining, using at least one machine learning algorithm, a presence of at least one abnormality associated with the one or more vessel segments based on the one or more computed tomography angiographic images.

According to various examples, the at least one machine learning algorithm may comprise one or more of an ensemble learning algorithm, a convolutional neural network, and a deep belief network.

Hereinafter, the processing of the one or more computed tomography angiographic images will be described in connection with ensemble learning as examples of the disclosure.

According to this disclosure, various ensemble learning algorithms can be used for determining the presence of the at least one abnormality, e.g., boosting including gradient boosting, extreme Gradient Boosting (XGBoost), and Adaptive Boosting (AdaBoost), bagging comprising random forest and Extra Trees classier, and stacking including super ensemble and blending techniques.

In general, the ensemble learning algorithm may be a parallel ensemble learning algorithm, a sequential ensemble learning algorithm, or a combination of both.

A parallel ensemble learning algorithm comprises multiple base machine learning models, e.g., classifiers or estimators, executed independently and combines respective outputs of the multiple base machine learning models using a combiner. The parallel ensemble learning algorithm may comprise bagging as well as its extension, and random forest algorithm. The parallel ensemble learning algorithm may be homogeneous or heterogeneous, depending on the base machine learning models' homogeneity. A homogeneous parallel ensemble learning algorithm consists of base machine learning models built using the same machine learning model, while heterogeneous ensembles comprise base machine learning models from different algorithms. The parallel ensemble learning algorithm may utilize the parallel connection of base machine learning models to encourage independence between the base machine learning models. For example, the independence of base machine learning models may significantly reduce the error due to the application of averages.

A sequential ensemble learning algorithm, e.g., AdaBoost, comprises multiple base machine learning models connected in a sequence. The sequential connection of the multiple base machine learning models may promote the dependence between the multiple base machine learning models. For example, the performance of the sequential ensemble learning algorithm may be improved by assigning higher weights to previously misrepresented base machine learning models.

According to various examples, the ensemble learning algorithm may comprise multiple base machine learning models and a combiner algorithm. Said determining of the presence of the at least one abnormality may comprise determining, using each of the multiple base machine learning models, a respective prediction of the presence of the at least one abnormality based on the one or more computed tomography angiographic images; and determining, using the combiner algorithm, the presence of the at least one abnormality based on the predictions respectively determined by the multiple base machine learning models. I.e., the ensemble learning algorithm may be a parallel ensemble learning algorithm.

According to various examples, the multiple base machine learning models may comprise a first base machine learning model. The method 3000 may further comprises determining, based on the one or more computed tomography angiographic images, centerlines of the one or more vessel segments, respectively. Each of the centerlines may comprise multiple points and each point is associated with a respective location of a respective vessel segment and a respective diameter of the respective vessel segment at the respective location. Said determining of the respective prediction of the presence of the at least one abnormality may comprise determining, using the first base machine learning model, a first prediction of the presence of the at least one abnormality based on the centerlines of the one or more vessel segments.

The centerlines of the one or more vessel segments can be determined using any one of existing centerline extraction techniques or by manual annotation. For example, such centerline extraction techniques may comprise techniques/methods presented or referred to in the following non-patent literature:
- Gtilstin, Mehmet A., et al. "Coronary centerline extraction via optimal flow paths and CNN path pruning." Medical Image Computing and Computer-Assisted Intervention-MICCAI 2016: 19th International Conference, Athens, Greece, October 17-21, 2016, Proceedings, Part III 19. Springer International Publishing, 2016.
- Schaap, Michiel, et al. "Robust shape regression for supervised vessel segmentation and its application to coronary segmentation in CTA." IEEE transactions on medical imaging 30.11 (2011): 1974-1986.
- Kirisli, H. A., et al. "Standardized evaluation framework for evaluating coronary artery stenosis detection, stenosis quantification and lumen segmentation algorithms in computed tomography angiography." Medical image analysis 17.8 (2013): 859-876.

According to various examples, before determining the first prediction of the presence of the at least one abnormality, the method 3000 may further comprise normalizing the respective diameter of the respective vessel segment at the respective location. As different patients with different gender and/or age may have quite varying diameter values of the respective vessel segment, the normalization of the respective diameter of the respective vessel segment can facilitate the first base machine learning model to determine the first prediction with higher accuracy.

For example, the normalization of the respective diameter of the respective vessel segment may be performed using Z-score normalization.

In general, Z-score normalization refers to the process of normalizing every value in a dataset such that the mean of all of the values is 0 and the standard deviation is 1.

According to various examples, the first base machine learning model may comprise a Recurrent Neural Network (RNN).

For example, the RNN may comprise those presented and referred to in non-patent literature - Michel, Anthony N. "Recurrent neural networks: overview and perspectives." Proceedings of the 2003 International Symposium on Circuits and Systems, 2003. ISCAS'03.. Vol. 3. IEEE, 2003.

Alternatively or optionally, the multiple base machine learning models may comprise a second base machine learning model. The method 3000 may further comprise determining, based on the one or more computed tomography angiographic images, one or more curved planar reformation (CPR) images depicting the one or more vessel segments. Said determining of the respective prediction of the presence of the at least one abnormality may comprise determining, using the second base machine learning model, a second prediction of the presence of the at least one abnormality based on the one or more curved planar reformation images.

The one or more CPR images may be determined using any one of existing techniques for determining or extracting CPR images. For example, such techniques for determining or extracting CPR images may comprise techniques/methods presented or referred to in the following non-patent literature:
- Gtilstin, Mehmet A., et al. "Coronary centerline extraction via optimal flow paths and CNN path pruning." Medical Image Computing and Computer-Assisted Intervention-MICCAI 2016: 19th International Conference, Athens, Greece, October 17-21, 2016, Proceedings, Part III 19. Springer International Publishing, 2016.
- Schaap, Michiel, et al. "Robust shape regression for supervised vessel segmentation and its application to coronary segmentation in CTA." IEEE transactions on medical imaging 30.11 (2011): 1974-1986.
- Kirisli, H. A., et al. "Standardized evaluation framework for evaluating coronary artery stenosis detection, stenosis quantification and lumen segmentation algorithms in computed tomography angiography." Medical image analysis 17.8 (2013): 859-876.

According to various examples, the one or more curved planar reformation images may comprise multiple curved planar reformation images respectively determined using different viewing directions. For example, the one or more CPR images may comprise multiple orthogonal CPR views, e.g., two, three, or four.

According to various examples, the second base machine learning model may comprise an encoder and a decoder. For example, the second base machine learning model may comprise those disclosed and referred to in non-patent literature - Yin, Xiao-Xia, et al. "U-Net-Based medical image segmentation." Journal of Healthcare Engineering 2022 (2022).

Alternatively or optionally, the multiple base machine learning models may comprise a third base machine learning model. The method 3000 may further comprise determining, based on the one or more computed tomography angiographic images, one or more images respectively depicting axial sections of the one or more vessel segments. Said determining of the respective prediction of the presence of the at least one abnormality may comprise determining, using the third base machine learning model, a third prediction of the presence of the at least one abnormality based on the one or more images respectively depicting the axial sections of the one or more vessel segments.

The one or more images respectively depicting axial sections of the one or more vessel segments may be determined using any one of existing vessel lumen segmentation techniques or by manual annotation. For example, such vessel lumen segmentation techniques may comprise techniques/methods presented or referred to in the following non-patent literature:
- Gtilstin, Mehmet A., et al. "Coronary centerline extraction via optimal flow paths and CNN path pruning." Medical Image Computing and Computer-Assisted Intervention-MICCAI 2016: 19th International Conference, Athens, Greece, October 17-21, 2016, Proceedings, Part III 19. Springer International Publishing, 2016.
- Schaap, Michiel, et al. "Robust shape regression for supervised vessel segmentation and its application to coronary segmentation in CTA." IEEE transactions on medical imaging 30.11 (2011): 1974-1986.
- Kirisli, H. A., et al. "Standardized evaluation framework for evaluating coronary artery stenosis detection, stenosis quantification and lumen segmentation algorithms in computed tomography angiography." Medical image analysis 17.8 (2013): 859-876.

According to various examples, each of the one or more images may be associated with semantic information related to the axial sections depicted in the respective image and said determining of the third prediction may be further based on the semantic information.

For example, the semantic information may comprise diameter values and/or locations of respective axial sections, and/or names of vessel segments where the respective axial sections are located.

According to various examples, the third base machine learning model may comprise a Convolutional Neural Network (CNN). For example, the third base machine learning model may comprise those disclosed and referred to in non-patent literature - Chauhan, Rahul, Kamal Kumar Ghanshala, and R. C. Joshi. "Convolutional neural network (CNN) for image detection and recognition." 2018 first international conference on secure cyber computing and communication (ICSCCC). IEEE, 2018.

Optionally or additionally, the method 3000 may comprise upon determining the presence of the at least one abnormality, determining a respective location and/or size of each of the at least one abnormality.

For example, at least one of the first, second, and third base machine learning models may label/classify/segment vessel points into two categories including abnormal vessel points and normal vessel points. The first, second, and/or third prediction of the presence of the at least one abnormality may comprise a respective count of abnormal vessel points and/or respective locations of the abnormal vessel points. The location and/or size of each of the at least one abnormality may be determined based on the respective locations of the abnormal vessel points.

Optionally or additionally, the method 3000 may comprise determining, based on the respective size, a respective Z-score for each of the at least one abnormality; and classifying, based on the respective Z-score, each of the at least one abnormality.

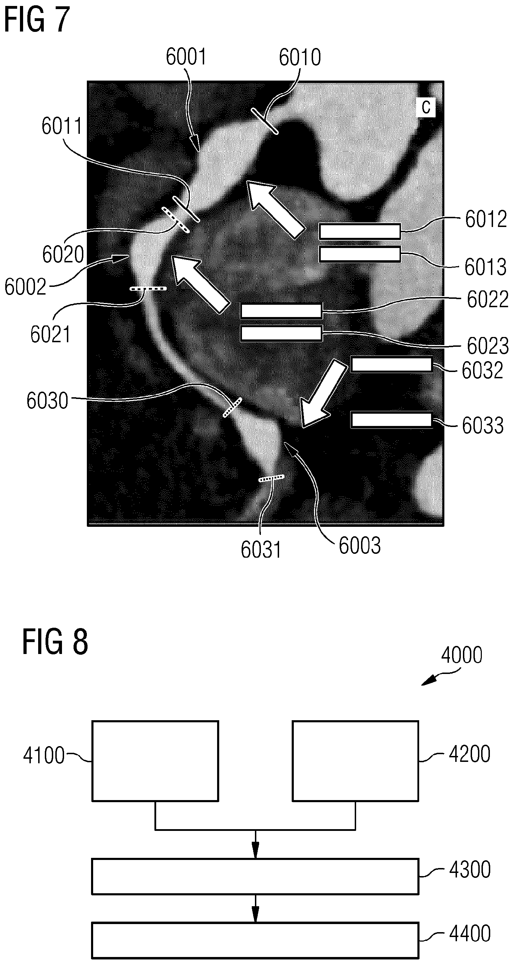

For example, FIG. 7 schematically illustrates an exemplary CTCA image depicting abnormalities with respective Z-scores and severity. Specifically, FIG. 7 shows respective locations, Z-scores, and severity of the aneurysms 6001, 6002, and 6003 as shown in the right image of FIG. 5.

As shown in FIG. 7, the location of the aneurysm 6001 may be indicated by a start point 6010 and an end point 6011. The severity 6012 and Z-score 6013 of the aneurysm 6001 may be also highlighted in the CTCA image.

Similarly, the location of the aneurysm 6002 may be indicated by a start point 6020 and an end point 6021. The severity 6022 and Z-score 6023 of the aneurysm 6002 may be also highlighted in the CTCA image.

Additionally or optionally, the location of the aneurysm 6003 may be indicated by a start point 6030 and an end point 6031. The severity 6032 and Z-score 6033 of the aneurysm 6003 may be also highlighted in the CTCA image.

According to this disclosure, location-wise (or point-wise) z-score can be calculated for all vessel locations (or points) of each vessel segment based on their vessel name and the luminal diameter value using one of the methods presented or referred to in a non-patent literature - Kim, Sung Hye. "Diagnosis of coronary artery abnormalities in Kawasaki disease: recent guidelines and z score systems." Clinical and Experimental Pediatrics 65.9 (2022): 430.

In preferred examples, Laura Olivieri's z-score calculation method may be used, which is disclosed in a non-patent literature - Olivieri, Laura, et al. "Coronary artery Z score regression equations and calculators derived from a large heterogeneous population of children undergoing echocardiography." Journal of the American Society of Echocardiography 22.2 (2009): 159-164.

After determining the respective Z-score for each of the at least one abnormality, e.g., aneurysms, classification of the respective abnormality can be performed, e.g., based on Table 2. The size of coronary artery abnormality may be classified according to internal lumen diameter, normalized for body surface area as Z-scores or standard deviation units.

**Table 2: Classification of coronary artery abnormalities**

| **Classification** | **Size of coronary artery abnormality** |
|---|---|
| No coronary involvement | Z-score always <2 and no more than a 0.9 decrease in Z-score during follow-up |
| Dilation only | Z-score 2 to <2.5 or if initially <2, a ≥1 decrease in Z-score during follow-up |
| Small aneurysm | Z-score ≥2.5 to <5 |
| Medium aneurysm | Z-score of ≥5 to <10 and absolute dimension <8 mm |
| Large aneurysm or giant aneurysm | Z-score ≥10 or absolute dimension ≥8 mm |

After determining the respective Z-score and severity classification for each of the at least one abnormality, the respective Z-score and severity may be displayed in an image like that shown in FIG. 7.

According to this disclosure, the ensemble learning algorithm can be trained using any one of the existing techniques, for example, as disclosed in a non-patent literature - Mienye, Ibomoiye Domor, and Yanxia Sun. "A survey of ensemble learning: Concepts, algorithms, applications, and prospects." IEEE Access 10 (2022): 99129-99149.

According to this disclosure, the method 3000 described above can be used for diagnosing a progression of at least one abnormality associated with one or more vessel segments based on CT angiographic images acquired during an exam and one or more follow-up exams of the anatomical region.

FIG. 8 is a flowchart of a further method 4000 according to various examples. The method 4000 pertains to establishing a diagnosis associated with one or more vessel segments comprised in an anatomical region of interest.

For example, the method 4000 may be used to determine a progression of one or more aneurysms located in a region of a heart of a child suffering from KD.

The method 4000 may be executed either during or after a CTA exam, e.g., a follow-up exam, of the anatomical region of interest, i.e., either real-time processing or post-processing is possible.

The method 4000 according to FIG. 8 may be executed by a computing device comprising at least one processing unit upon loading program code from a memory. Such a computing device may be either embedded in a CT scanner such as the CT scanner 1000 shown in FIG. 1 or connectable to the CT scanner or a PACS. Details of the method 4000 are described below.

Block 4100: obtaining a first set of computed tomography angiographic images acquired during an exam of an anatomical region of interest comprising one or more vessel segments.

According to various examples, the first set of computed tomography angiographic images may be acquired during a previous exam of the anatomical region of interest and stored in a database, e.g., a PACS.

Block 4200: obtaining a second set of computed tomography angiographic images acquired during a follow-up exam of the anatomical region of interest.

According to various examples, the second set of computed tomography angiographic images may be obtained directly from a CT scanner such as the CT scanner 1000 shown in FIG. 1 or from a database for storing the one or more CT angiographic images acquired by the CT scanner, e.g., a PACS.

I.e., the method 4000 may be executed during or after the follow-up exam.

According to this disclosure, blocks 4100 and 4200 can be executed either in parallel or sequentially.

Block 4300: processing the first set and the second set according to the method 3000 described above, respectively.

According to various examples, the first set of the computed tomography angiographic images may be processed using the method 3000 during or after the exam of the anatomical region of interest and the result of the processing may be stored in a database, e.g., the PACS.

Alternatively or optionally, the first set of the computed tomography angiographic images may be processed using the method 3000 during or after the follow-up exam of the anatomical region of interest.

Block 4400: establishing a diagnosis associated with the one or more vessel segments based on said processing.

According to various examples, the diagnosis may comprise a presence of one or more abnormalities, an increase of size of one or more abnormalities, and/or an increasing severity of the one or more abnormalities. The diagnosis may be established by comparing respective results of the processing of the first set and the second set of the computed tomography angiographic images.

For example, referring back to Fig. 7, based on the first set of the computed tomography angiographic images, the Z-score and severity of the aneurysm 6001 are determined to be 7.1 and Medium aneurysm, respectively, while based on the second set of the computed tomography angiographic images, the Z-score and severity of the aneurysm 6001 are determined to be 11.2 and Giant aneurysm, respectively. Accordingly, the diagnosis can be established as an increasing severity of the aneurysm 6001.

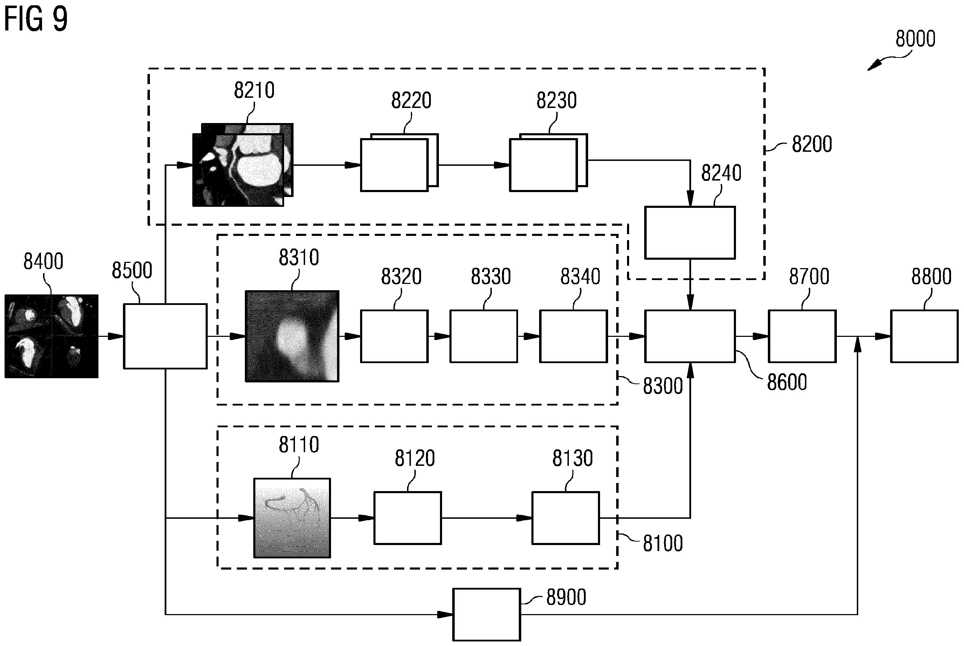

FIG. 9 schematically illustrates an exemplary workflow 8000 according to various examples. The workflow 8000 pertains to processing CT angiographic images to determine, detect, or diagnose one or more aneurysms associated with one or more vessel segments based on the method 3000. I.e., the workflow 8000 may be used for automatic detection and severity classification of Coronary Artery Aneurysms.

The workflow 8000 may be executed either during or after a CTA exam of the heart of a patient, i.e., either real-time processing or post-processing is possible.

The workflow 8000 may be executed by a computing device comprising at least one processing unit upon loading program code from a memory. Such a computing device may be either embedded in a CT scanner such as the CT scanner 1000 shown in FIG. 1 or connectable to the CT scanner or a PACS. Details of the workflow 8000 are described below.

### Automatic detection of Coronary Artery Aneurysms

As shown in FIG. 9, the input CTCA images 8400 may be first processed using Cardiac Segmentation and Coronary Tracing algorithms 8500 such as those disclosed and referred to in the following non-patent literature:
- Gtilstin, Mehmet A., et al. "Coronary centerline extraction via optimal flow paths and CNN path pruning." Medical Image Computing and Computer-Assisted Intervention-MICCAI 2016: 19th International Conference, Athens, Greece, October 17-21, 2016, Proceedings, Part III 19. Springer International Publishing, 2016.
- Schaap, Michiel, et al. "Robust shape regression for supervised vessel segmentation and its application to coronary segmentation in CTA." IEEE transactions on medical imaging 30.11 (2011): 1974-1986.
- Kirisli, H. A., et al. "Standardized evaluation framework for evaluating coronary artery stenosis detection, stenosis quantification and lumen segmentation algorithms in computed tomography angiography." Medical image analysis 17.8 (2013): 859-876.

Then, outputs of the Cardiac Segmentation and Coronary Tracing algorithms 8500 may be respectively processed following three paths 8100, 8200, and 8300, for aneurysm location detection, then respective results of the three paths 8100, 8200, and 8300 may be combined using an ensemble learning algorithm, i.e., a combiner of the ensemble learning algorithm. Then, the location and extent of aneurysms 8700 may be precisely determined, detected, or predicted.

Additionally or optionally, a severity of each detected aneurysm may be classified based on the respective location and extent of aneurysms 8700.

The Cardiac Segmentation and Coronary tracing algorithms 8500 may give coronaries with semantics containing diameter values at resampled vessel points and detect several centerlines, where a centerline is a vessel path starting from the aorta to the extreme distal end of a vessel.

Following are the details of each of the algorithmic paths 8100, 8200, and 8300 for aneurysm detection:
Path 8100: Sequence-to-sequence-based path with vessel diameter values
   Block 8110: Vessel points on the centerlines along with their diameter values may be received through the cardiac segmentation and coronary tracing algorithms 8500;
   Block 8120: Received diameter values may be then normalized so that the model predicts aneurysms with higher accuracy even for different patients, as diameter values might be quite varying for different patients.
   In the preferred embodiment, the established z-score normalization (z-score = (point Value - mean Value)/standard-deviation) may be used.
   Block 8130: These normalized values may be fed to a sequence-to-sequence prediction model as a series and the model labels different points on the vessel whether it belongs to an aneurysm. The output of the model may comprise the vessels with their points labeled if they belong to an aneurysm.
   In preferred examples, the model comprises an RNN.
Path 8200: Segmentation-based path with CPR views
   Block 8210: CPR images/views for different centerlines may be produced after cardiac segmentation and coronary tracing.
   Block 8220: The CPR images/views may be then fed into a segmentation model to generate a semantic segmentation for aneurysms.
   In preferred examples, the segmentation model may comprise a U-Net-based machine learning model.
   Block 8230: From the segmentation generated at block 8220, the vessel points may be labeled as whether they belong to an aneurysm.
   Block 8240: Labeled results from different CPR views for a centerline may be then adjudicated to give final labeled results.
   The output of path 8200 may comprise the vessels with their points labeled if they belong to an aneurysm.
Path 8300: Path with cross-sectional views
   Block 8310: Cross-sectional views of the vessel at different tree points may be produced after cardiac segmentation and coronary tracing algorithms are run.
   Those views (stack of images) may be fed into a prediction model that labels each lumen cross-section describing whether it belongs to an aneurysm based on the input cross-section image and its other vessel point semantic values like vessel name, distance from corresponding ostia point, etc.
   In preferred examples, the model may comprise a CNN where the image may be first fed to a convolution layer at block 8320 and the output of this layer may be then combined with semantic values (vessel name, distance from Ostia, etc.) and fed to the rest part of the model at block 8330.
   Block 8340: the model may output the vessels with their points labelled if they belong to an aneurysm.
Block 8600: The results output from the three paths 8100, 8200, and 8300 may be then combined using an Ensemble Learning algorithm to determine, detect, or predict final labeling for the vessel points.

Once the final labeling of vessel points is done, these labels can then be aggregated to determine, detect, or predict the final location and extent of different aneurysms.

### Z-score calculation of vessel points' diameter values

Block 8900: location-wise (or point-wise) z-score can be calculated for all vessel locations (or points) of each vessel segment based on their vessel name and the luminal diameter value using one of the methods presented or referred to in a non-patent literature - Kim, Sung Hye. "Diagnosis of coronary artery abnormalities in Kawasaki disease: recent guidelines and z score systems." Clinical and Experimental Pediatrics 65.9 (2022): 430.

In preferred examples, Laura Olivieri's z-score calculation method may be used, which is disclosed in a non-patent literature - Olivieri, Laura, et al. "Coronary artery Z score regression equations and calculators derived from a large heterogeneous population of children undergoing echocardiography." Journal of the American Society of Echocardiography 22.2 (2009): 159-164.

### Severity classification of Coronary Artery Aneurysms

Block 8800: After determining the respective Z-score for each of the aneurysms, classification of the respective aneurysms can be performed, e.g., based on Table 2. The size of coronary artery aneurysms may be classified according to internal lumen diameter, normalized for body surface area as Z-scores or standard deviation units.

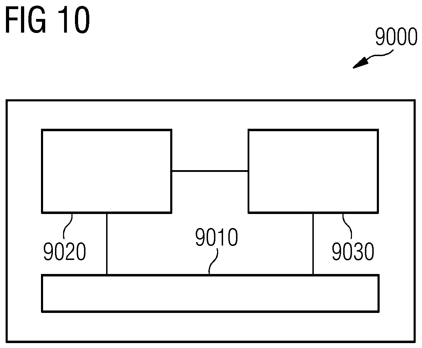

FIG. 10 is a block diagram of a computing device 9000 according to various examples. The computing device 9000 pertains to determining, detecting, or diagnosing a presence of at least one abnormality associated with one or more vessel segments comprised in an anatomical region of interest based on the method 3000 and/or establishing a diagnosis associated with one or more vessel segments comprised in an anatomical region of interest based on the method 4000.

The computing device 9000 may comprise a processor 9020, a memory 9030, and an input/output interface 9010. The processor 9020 is configured to load program code from the memory 9030 and execute the program code. Upon executing the program code, the processor 9020 performs the method 3000 and/or the method 4000, respectively.

Referring to FIG. 1 again, the CT scanner 1000 may comprise a computing unit configured to perform the method 3000 and/or 4000.

Alternatively, the computing device 9000 may be embedded in or connected with the CT scanner 1000, and thereby the CT scanner 1000 may be also configured to perform the methods 3000 and/or 4000.

Summarizing, techniques have been described that facilitate diagnosing abnormalities associated with one or more vessel segments within an anatomical region of interest, e.g., during the acute stage and/or on follow-up. By processing CT angiographic images using at least one machine learning algorithm, e.g., an ensemble learning algorithm, a presence of at least one abnormality associated with the one or more vessel segments can be automatically, precisely, reliably, and robustly determined, detected, predicted, or diagnosed. Further, the proposed techniques can capture the superficial vessels with high accuracy, thereby being able to detect deep-lying abnormalities, e.g., aneurysms, in the vessels which might get missed in the echocardiography diagnosis. The disclosed techniques can make use of machine-learning-based algorithms to simplify decision-making and enable less experienced radiologists to perform the diagnosis. The 3D nature of the CT images may further reduce human error by providing post-processing techniques to highlight and auto-detect the aneurysms. When there is a need for a follow-up exam in order to determine the progression of the Coronary Artery Aneurysms and monitor the development of thrombotic and steno-occlusive complications. CTCA with modern scanners may allow highly accurate image registration algorithms, thereby making the follow-up much more precise.

Although the disclosure has been shown and described with respect to certain preferred embodiments, equivalents and modifications will occur to others skilled in the art upon the reading and understanding of the specification. The present disclosure includes all such equivalents and modifications and is limited only by the scope of the appended claims.

For illustration, the disclosure is explained in detail based on CTCA, the techniques disclosed herein can be also applied to CT angiographic images of the brain, the kidney, or the lung.

## Claims

1. A computer-implemented method (3000), comprising:
- obtaining (3100) one or more computed tomography angiographic images associated with an anatomical region of interest, wherein the anatomical region of interest comprises one or more vessel segments (201-218);
- determining (3200), using at least one machine learning algorithm, a presence of at least one abnormality (6001, 6002, 6003) associated with the one or more vessel segments (201-218) based on the one or more computed tomography angiographic images.

2. The computer-implemented method (3000) of claim 1,
wherein the anatomical region of interest comprises a region of a heart, in particular a region of a heart of a child, and the at least one abnormality (6001, 6002, 6003) comprises one or more aneurysms.

3. The computer-implemented method (3000) of claim 1 or 2,
wherein the at least one machine learning algorithm comprises an ensemble learning algorithm.

4. The computer-implemented method (3000) of claim 3,
wherein the ensemble learning algorithm comprises multiple base machine learning models and a combiner algorithm;
said determining of the presence of the at least one abnormality (6001, 6002, 6003) comprises:
- determining, using each of the multiple base machine learning models, a respective prediction of the presence of the at least one abnormality (6001, 6002, 6003) based on the one or more computed tomography angiographic images;
- determining, using the combiner algorithm, the presence of the at least one abnormality (6001, 6002, 6003) based on the predictions respectively determined by the multiple base machine learning models.

5. The computer-implemented method (3000) of claim 4,
wherein the multiple base machine learning models comprises a first base machine learning model;
the method further comprising:
- determining, based on the one or more computed tomography angiographic images, centerlines of the one or more vessel segments (201-218), respectively, wherein each of the centerlines comprises multiple points and each point is associated with a respective location of a respective vessel segment (201-218) and a respective diameter of the respective vessel segment (201-218) at the respective location;
said determining of the respective prediction of the presence of the at least one abnormality (6001, 6002, 6003) comprises:
- determining, using the first base machine learning model, a first prediction of the presence of the at least one abnormality (6001, 6002, 6003) based on the centerlines of the one or more vessel segments (201-218) .

6. The computer-implemented method (3000) of claim 5, further comprising:
- normalizing the respective diameter of the respective vessel segment (201-218)at the respective location.

7. The computer-implemented method (3000) of any one of claims 4 - 6, wherein the multiple base machine learning models comprises a second base machine learning model;
the method further comprising:
- determining, based on the one or more computed tomography angiographic images, one or more curved planar reformation images depicting the one or more vessel segments (201-218);
said determining of the respective prediction of the presence of the at least one abnormality (6001, 6002, 6003) comprises:
- determining, using the second base machine learning model, a second prediction of the presence of the at least one abnormality (6001, 6002, 6003) based on the one or more curved planar reformation images.

8. The computer-implemented method (3000) of claim 7, wherein the one or more curved planar reformation images comprise multiple curved planar reformation images respectively determined using different viewing directions.

9. The computer-implemented method (3000) of any one of claims 4 - 8, wherein the multiple base machine learning models comprises a third base machine learning model;
the method further comprising:
- determining, based on the one or more computed tomography angiographic images, one or more images respectively depicting axial sections of the one or more vessel segments (201-218);
said determining of the respective prediction of the presence of the at least one abnormality (6001, 6002, 6003) comprises:
- determining, using the third base machine learning model, a third prediction of the presence of the at least one abnormality (6001, 6002, 6003) based on the one or more images respectively depicting the axial sections of the one or more vessel segments (201-218).

10. The computer-implemented method (3000) of claim 9,
wherein each of the one or more images is associated with semantic information related to the axial sections depicted in the respective image and said determining of the third prediction is further based on the semantic information.

11. The computer-implemented method (3000) of any one of the preceding claims, further comprising:
- upon determining the presence of the at least one abnormality (6001, 6002, 6003), determining a respective location and/or size of each of the at least one abnormality (6001, 6002, 6003).

12. The computer-implemented method (3000) of claim 11, further comprising:
- determining, based on the respective size, a respective Z-score for each of the at least one abnormality (6001, 6002, 6003); and,
- classifying, based on the respective Z-score, each of the at least one abnormality (6001, 6002, 6003).

13. A computer-implemented method, comprising:
- obtaining (4100) a first set of computed tomography angiographic images acquired during an exam of an anatomical region of interest comprising one or more vessel segments (201-218);
- obtaining (4200) a second set of computed tomography angiographic images acquired during a follow-up exam of the anatomical region of interest;
- processing (4300) the first set and the second set according to the method of any one of the preceding claims, respectively;
- establishing (4400) a diagnosis associated with the one or more vessel segments (201-218) based on said processing.

14. A computing device (9000), the device comprising a processor (9020) and a memory (9030), wherein upon loading and executing program code from the memory (9030), the processor (9020) is configured to perform the method of any one of the preceding claims.

15. A computed tomography scanner (1000) comprising the computing device (9000) of claim 14.
